(19) Europäisches Patentamt / European Patent Office / Office européen des brevets

(11) **EP 3 199 119 A1**

(12) **EUROPEAN PATENT APPLICATION**

(43) Date of publication:
**02.08.2017 Bulletin 2017/31**

(51) Int Cl.:
***A61B 18/12*** *(2006.01)*

(21) Application number: **17151046.4**

(22) Date of filing: **11.01.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.01.2016 US 201662279061 P**

(71) Applicant: **Cook Medical Technologies LLC Bloomington, IN 47404 (US)**

(72) Inventors:
• **ELGAARD, Per**
  **4690 Haslev (DK)**
• **PAAMAND, Rune Tore**
  **3050 Humlebaek (DK)**

(74) Representative: **Garratt, Peter Douglas et al Mathys & Squire LLP The Shard 32 London Bridge Street London SE1 9SG (GB)**

(54) **MEDICAL DEVICE**

(57) The present disclosure provides for a device and methods of use to endoluminally ablate and/or occlude a body vessel using a radiofrequency ("RF") signal and a current to perform resistive heating. The device may have a RF mode and a resistive mode, and may perform each mode in sequential order until the vessel is fully occluded. The device further comprises a control unit to operate the device in its various modes.

FIG. 3

EP 3 199 119 A1

**Description**

CROSS-REFERENCE TO RELATED APPLICATIONS

This application claims the benefit of U.S. Provisional Application No. 62/279,061, filed on January 15, 2016.

FIELD

The present disclosure relates generally to medical devices. More specifically, the disclosure relates to a device and method(s) for occluding or closing a body vessel using a radiofrequency signal and a current to heat and/or ablate the body vessel.

BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.
Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.
It is also known to seek to constrict a vessel by endoluminal ablation, causing contraction of the vessel and/or coagulation of blood to form a blood clot in the vessel. Various methods can be employed to cause such ablation.

BRIEF SUMMARY

The invention may include any of the following embodiments in various combinations and may also include any other aspect described below in the written description or in the attached drawings. This disclosure provides a medical device and methods for conducting vessel ablation and occlusion.
The device may be used to heat a body vessel, and it may have a support including a proximal end and extending to a distal end, the support defining a longitudinal axis. The device may optionally include a coil disposed about the support and being electrically conductive. The coil may have a first end being disposed between the proximal and distal ends, the coil extending from the first end to a second end disposed at the distal end.
The device may further include a control unit being operatively coupled to the first end and the second end, the control unit having a signal generator and a power supply. The signal generator may be operable to transmit a radiofrequency signal from the coil to the body vessel when the device is in a radiofrequency mode. The resistive part may be at a first temperature in the radiofrequency mode. The power supply may be operable to transmit a current through the coil when the device is in a resistive mode. The current may heat the resistive part to a second temperature being greater than the first temperature in the resistive mode. The second temperature may be sufficient to occlude and swell the body vessel.
The device may be part of an assembly having the features discussed above, and also having an outer sheath having a first sheath end and extending to a second sheath end. The outer sheath may form an inner lumen therethrough from the first sheath end to the second sheath end such that the support is slidably disposed within the inner lumen.
Various additional features and embodiments will become apparent with the following description. The present disclosure may be better understood by referencing the accompanying figures.

BRIEF DESCRIPTION OF THE DRAWINGS

Fig. 1 depicts a partial, environmental view of a medical device in accordance with one embodiment of the present disclosure;
Fig. 2A depicts a cross-sectional, side view of the device of Fig. 1;
Fig. 2B depicts a partial, blown-up, side view of the device of Fig. 1;
Fig. 3 depicts a side view of the device of Fig. 1;
Fig. 4 depicts a side view of the device of Fig. 1 in a radiofrequency mode;
Fig. 5 depicts a side view of the device of Fig. 1 in a resistive mode; and

Fig. 6 depicts steps of a method of use of the device of Fig. 1 in accordance with one embodiment of the present disclosure.

DETAILED DESCRIPTION

The present disclosure will now be described more fully with reference to the accompanying figures, which show various embodiments. The accompanying figures are provided for general understanding of various embodiments and method steps. However, this disclosure may be embodied in many different forms. These figures should not be construed as limiting, and they are not necessarily to scale.

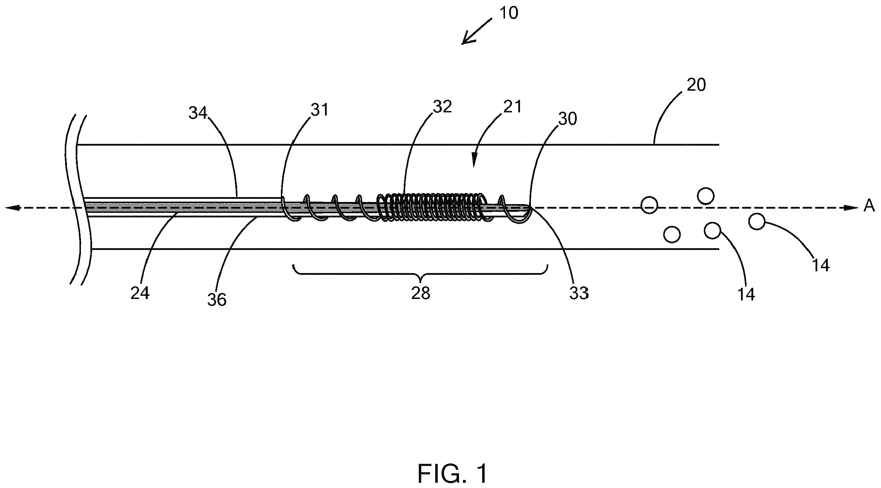

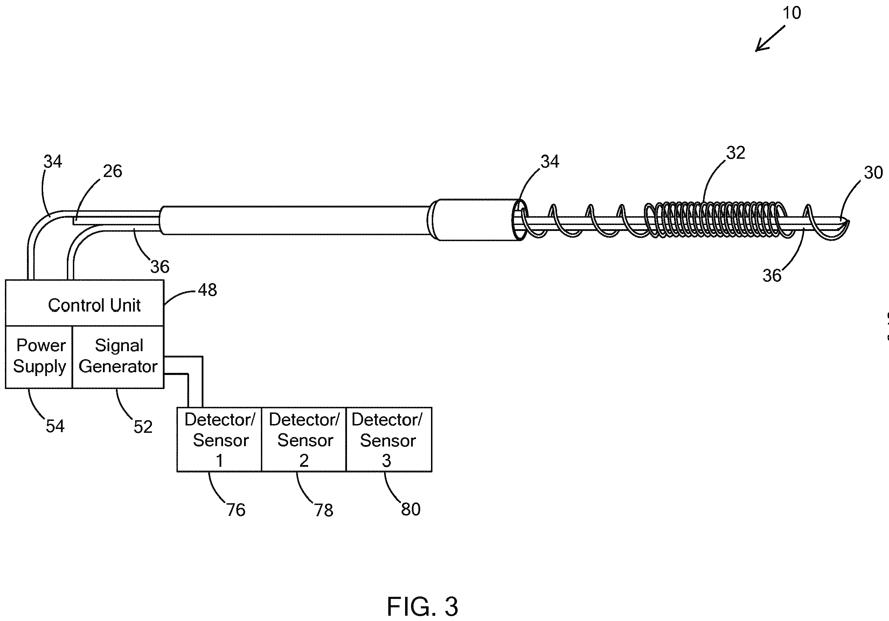

Fig. 1 depicts an environmental view of one embodiment of the medical device 10 that may be used to heat a body vessel at or adjacent a treatment site 21. The body vessel has a vessel wall 20. In this view, the device 10 may be placed or positioned in the body vessel in a body. The device 10 may have a support 24, or mandrel, that extends from a proximal end (depicted in Fig. 3 (26)) to a distal end 30. The support 24 may define a longitudinal axis A.

A coil 32 may be disposed about the support 24, and the coil 32 may be electrically conductive and exposed to the surrounding environment. The coil 32 may have a first end 31 being disposed between the proximal and distal ends (26, 30), and the coil 32 may extend to a second end 33 being disposed at the distal end 30. The support 24 may have a distal segment 28 that supports the coil 32, or about which the coil 32 is disposed.

The coil may give the advantage of being able to deliver more energy and power to the vessel and surrounding environment, especially when compared to a straight electrode tip. The coil may also make better contact with the vessel wall during use.

The distal segment 28 may have an outer diameter that distally decreases to form a distal taper (as shown in Fig. 1). Alternatively, the support 24, including its distal segment 28, may have an outer diameter being uniform from the proximal end 26 to the distal end 30. The distal taper or tapered tip may provide the advantage of making the distal end 30 easier to track within the body vessel. Such distal taper may provide flexibility to track the device 10. Additionally, the device may have a curvature at the distal taper region or tip to facilitate tracking through the vasculature, such as may be seen in common guidewires.

The device 10 may further have a first wire 34 and a second wire 36. The first wire 34 may be electrically coupled or connected to a control unit (depicted in Fig. 3 (48)). The first wire 34 may extend from the control unit and along the longitudinal axis A to the first end 31. The first wire 34 may be attached to the first end 31 such that the connection provides an electrical coupling between the first wire 34 and the coil 32.

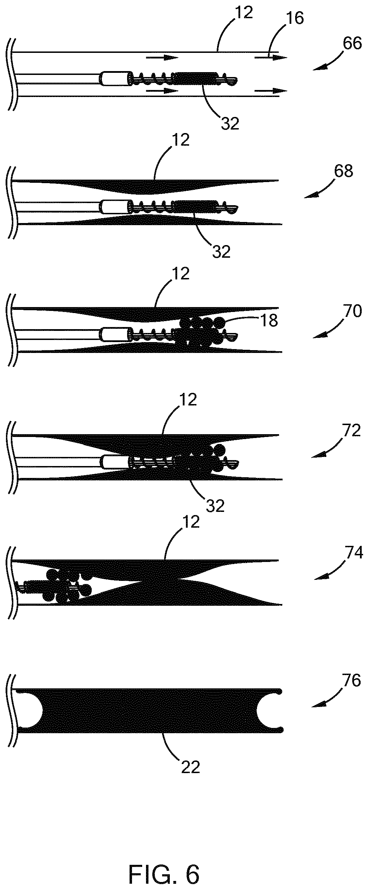

The second wire 36 may also be electrically coupled or connected to the control unit and extend from the control unit, along the longitudinal axis A, to the second end 33. The second wire 36 may be attached to the second end 33 such that the connection provides an electrical coupling between the second wire 36 and the coil 32. In this way, the device 10 may form a first circuit along the path created by the control unit, the first wire 34, the coil 32, and the second wire 36. The device 10 may form various circuits, which will be discussed further in Figs. 3-5. In Fig. 1, coagulated blood 14 may start to form as the device 10 operates, which will also be discussed further in Fig. 6.

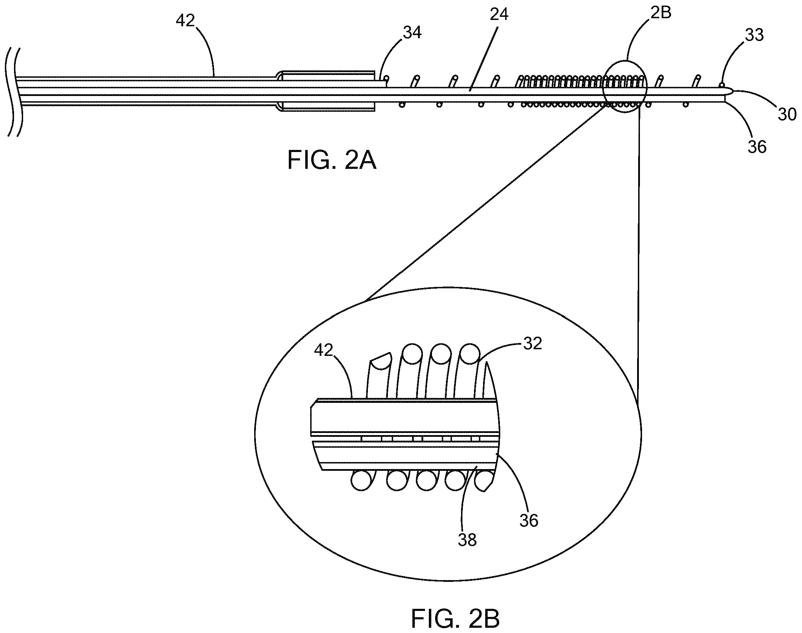

Figs. 2A-B depicts further details of the device. For example, Fig. 2A shows a cross-sectional view of the device. Fig. 2B shows a blown-up view of the device around circle 2B. In Fig. 2A, the support 24 has a uniform outer diameter from the proximal end to the distal end 30. Additionally, either or both of the first and second wires (34, 36) may have an insulator disposed about their outer surface to electrically insulate or isolate them from the rest of the device. Insulator 38 is disposed about the second wire 36 in Fig. 2B.

Additionally, the device may have a shrink tubing disposed about the device. For example in Fig. 2A, the shrink tubing 42 may extend around the support 24, the first wire 34, the second wire 36, and/or any other portion of the device. As one advantage, the shrink tubing 42 may immobilize or bind the support 24, the first wire 34, and the second wire 36 in place so that they are immobilized relative to each other. The shrink tubing 42 may also immobilize and/or extend over a part of the coil 32 to keep the coil 32 in position as the device is in use. Alternatively or additionally in Fig. 2B, the shrink tubing 42 may only be disposed about the support 24. In this configuration, the shrink tubing 42 may act to isolate or insulate the support 24 from the rest of the device. The coil itself may have at least some part being electrically exposed.

Fig. 3 further depicts the proximal end of the device 10. The proximal end may include a control unit 48 being operatively coupled or connected to the first end of the coil 32 by way of the first wire 34. Additionally, the control unit 48 may be operatively coupled to the second end of the coil 32 by way of the second wire 36. The control unit 48 may be coupled to or include a signal generator 52 to generate a radiofrequency signal and a power supply 54 to generate a current. The power supply could be a battery and/or other forms of direct current and/or alternating current.

The signal generator 52 may be operable by the control unit 48 and the user to transmit the RF signal from the coil and to the body vessel when the device 10 is in a radiofrequency mode. This RF signal may be a AC signal from about 10kHz to about 1 MHz. The power supply 54 may be operable by the control unit 48 and the user to transmit a current through the coil when the device 10 is in a resistive mode. In the resistive mode, the current may be a direct current and/or an alternating current.

In either mode, the amount of current may vary over time, and may depend on the required power to maintain a desired temperature of the coil.

$$\text{Power} = \text{Current} \times \text{Voltage} \qquad \text{Power} = \text{Current}^2 \times \text{Resistivity}$$

A device that operates in both RF and resistive heating modes may start with a resistivity of about 50 ohms and a current of about 0.50 amps in the RF mode. After charring occurs, the conductance of the coil will be limited. At this point, the device may switch into resistive heating mode, having a resistivity of about 120 ohms and a current of about 0.33 amps. Charring on the coil may create an increase in overall resistance and/or impedance of the electrical circuits in the device.

In the RF mode, the RF signal generates a field around the tip. The RF signal may not deliver enough power to heat the coil itself. Rather, the coil is maintained at a first temperature (e.g. body temperature). In the resistive mode, the power delivered may increase and be great enough to heat the resistive part (e.g. resistive tip at second end 33) to a target temperature, or second temperature being greater than the first temperature, such that the target temperature is sufficient to inflame or occlude the body vessel. In this way, the coil may be at a first temperature in RF mode and at a second, higher temperature in the resistive heating mode. The second temperature may be just above human body temperature or much greater than human body temperature. These modes of the device, and how they contribute to heating the body vessel, will be discussed in further detail below.

The control unit 48 may also include an electrode drive unit (not shown) for moving the coil within the patient's vessel. This may also be done manually. In some embodiments, the control unit 48 may have or be coupled to a plurality of sensors and/or detectors (76, 78, 80) to determine different conditions of the device 10. For example, the plurality of sensors may be sensors selected from the group consisting of temperature sensors, current sensors, timers, impedance/resistance sensors, and pressure sensors. These various sensors may be used to detect and determine temperature, current, time, impedance, and/or resistance, respectively, to assist the user in using the device 10 as the active components may not be visually accessible to the user.

The control unit 48 may optionally include a user interface coupled to the control unit 48 and operable to provide data to the user and for input of user commands to the control unit 48. The user interface may, in its simplest embodiment, include an on/off switch for operating the control unit 48, and ablation and/or coagulation, with the control unit 48 then effecting the desired ablation process under the command of the control unit 48. In other embodiments, the user interface may be more sophisticated and enable, for example, a user to select different modes of ablation and optionally to produce, for instance, occluding barriers of different lengths and/or different sizes.

The user interface also may have an output for providing ablation feedback and/or warning signals to a user. It may, for example, provide an indication of measured temperature and/or impedance, an indication of progress of ablation of the vessel and so on. For such purposes, the user interface may include a visual unit, for example a display to display quantitative data such as graphs, measures of temperature and impedance, determined length of occlusion and so on. In other embodiments, the display may be simpler, having for instance simple visual indicators such as one or more illuminated lamps. The output could also be an acoustic output and/or, as appropriate, a tactile output such as a vibration generator and so on. Any combination of user feedback devices may be provided.

When in use, the device may operate in two modes: (1) a RF mode for RF heating and/or ablation and (2) a resistive heating mode for resistive heating and ablation. Both modes may use the coil 32 to create a closed loop or circuit. In one or both modes, the device is designed to create blood clotting, that is to ablate the blood surrounding the electrical element. This can be achieved by selecting an ablation energy level and an ablation time duration suitable to heat surrounding blood, which in some circumstances can be expected to be less than the energy required to ablate the vessel tunica (e.g. tunica externa), although there may be experienced some contraction of the vessel as a result of the heating of the blood. The skilled person will be able to determine suitable ablation parameters from common general knowledge in the art.

It is to be appreciated that the level of power applied through the electrode and the time of application will be dependent upon factors including the size of the vessel, the amount and speed blood flow through the vessel, pulsation and turbulence of blood at the point of ablation, and so on.

In Fig. 3, elements of the device 10 may create various circuits. For example, Fig. 3 depicts elements that form a first circuit or electrical pathway for use in the resistive mode. Specifically, the first wire 34 may be electrically coupled to the control unit 48 and extend from the control unit 48 and along the longitudinal axis to the first end of the coil 32. The first wire 34 may be attached to the first end of the coil 32. The second wire 36 may also be electrically coupled to the control unit 48 and extend from the control unit 48 and along the longitudinal axis to the second end of the coil 32. The second wire 36 may be attached to the second end of the coil 32. The control unit 48, the first wire 34, the coil 32, and a second wire 36 form the first circuit being operable in the resistive mode. Further details of the RF and resistive modes will be discussed below in turn.

**RF Mode**

Generally in the RF mode, an electrical terminal is fed endoluminally into the vessel and an electrical pulse and/or constant electrical signal at RF frequencies is applied to the electrical terminal. The conductivity of blood and/or the vessel tissues causes localized heating of the blood and tissue and not significant heating of the resistive part of the coil itself, creating a local zone that is heated by the RF frequencies. This heating can be used to cause damage to the tissue (intima) of the vessel wall, resulting in vessel contraction. In other devices, RF ablation heats the surrounding blood, causing this to coagulate around the electrical terminal and form a blood clot which blocks the vessel.

Two types of RF ablation apparatus are generally contemplated in the art: a monopolar system and a bipolar system. A monopolar system may have an elongate first electrode (e.g. active electrode) and a second electrode (e.g. dispersive electrode) pad or return electrode positioned outside the patient's body. The first electrode is designed to be fed endoluminally into the patient's vessel, while the second electrode pad is positioned against the person's outer body, as close as practicable to the first electrode. Electrical energy applied to the first electrode will pass by conduction through the patient to the pad. There will be localized heating at the first electrode, which effects the desired ablation. The RF frequencies, and the power they generate, may not be large enough to heat the first electrode and/or the return electrode. However, the RF signal and field generated will cause localized heating of the surrounding blood and tissue. The temperature of the first electrode remains at a first temperature (e.g. body temperature).

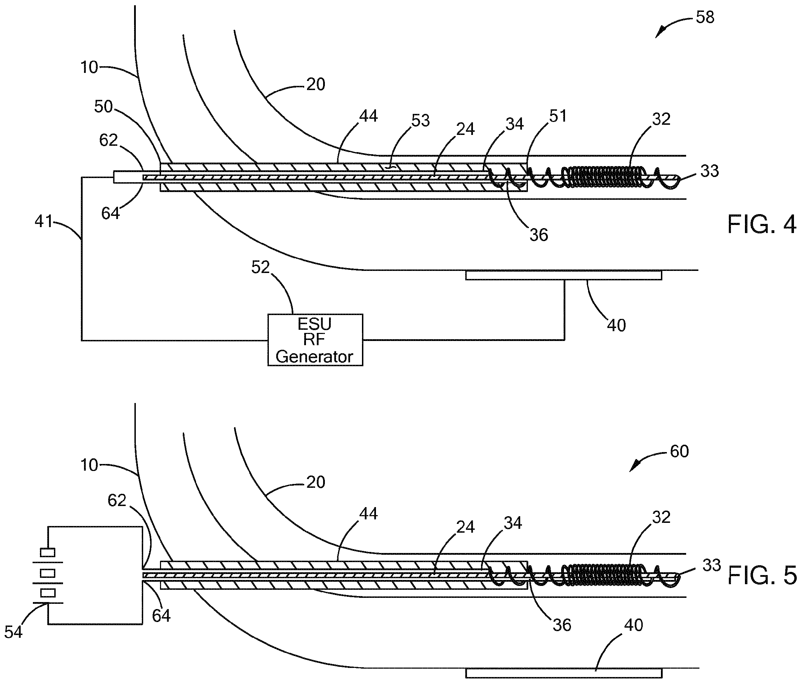

Fig. 4 depicts details of the device in the RF mode 58. A pad or return electrode 40 may be arranged in various forms having various circuits. For example, in a monopolar system or mode, the return electrode 40 may be electrically coupled to the control unit (e.g. RF generator 52) and disposable outside of the body 10. In this example, the RF signal generator 52, the first wire 34, the coil 32, and return electrode 40 may form a second circuit for the RF signal.

In the monopolar mode, the radiofrequency signal may be transmitted through the second circuit having the RF signal generator 52, the first wire 34, the coil 32, and the second wire 36 including the return electrode 40. Advantageously, the monopolar system may be easier to manufacture, having less elements immobilized or attached to the support 24.

The RF mode 58 may also operate in a bipolar system. In a bipolar system, the return electrode 40 may be disposed within the second wire 36 (similarly to the first circuit of the resistive mode discussed with Fig. 3). In this way, the signal generator 52, the first wire 34, the coil 32, and a return electrode 40 being part of the second wire 36 form a third circuit for the RF signal.

In the bipolar mode, the radiofrequency signal may be transmitted through the third circuit having the RF signal generator 52, the first wire 34, the coil 32, and the second wire 36. Advantageously, operating the device having a bipolar system may be easier for the user by not having a separable return electrode disposable outside of the body 10.

Using either the second circuit (monopolar system) or the third circuit (bipolar system), the device may transmit an RF signal from the coil 32 to the return electrode 40, heating the body vessel when in the RF mode. A method step of transmitting a RF signal may include transmitting the RF signal with the monopolar mode or the bipolar mode.

The first wire 34 may be attached via a first lead 62 and a conductive wire 41 to the signal generator 52. Additionally, the second wire 36 may be attached to the signal generator 52 through a second lead 64 and the conductive wire 41. One skilled in the art will understand that various conductive wires and connectors may be used to electrically couple parts of the device.

The device may optionally include an outer sheath 44 for delivery and/or retrieval. The outer sheath 44 may optionally have a first sheath end 50 and extend to a second sheath end 51. The outer sheath 44 may form an inner lumen 53 therethrough from the first sheath end 50 to the second sheath end 51. As shown in Fig. 4, the support 24 may be slidably disposed or received within the inner lumen 53.

**Resistive Heating Mode**

The resistive part of the coil 32 has a higher electrical resistance than the other parts of the electrically conductive element (e.g. 50-200 ohms). In this embodiment, the resistive part is the operative part of the device. The resistive part is configured so that the application of power to the wires (34, 36) causes current to flow through the resistive part, which can cause heating of the resistive part to a second temperature being greater than body temperature. This, in turn, causes embolization and/or ablation of blood surrounding the resistive part, and/or heating and consequential contraction of the vessel in the vicinity of the resistive part through the thermal energy at the resistive part. Structurally, the resistive part of the coil could be any part of the coil. In one example, the resistive part is the second end 33.

Fig. 5 depicts the resistive heating mode or resistive mode 60. In a method of use of the device, after transmitting the RF signal, the control unit and/or a sensor may detect at least one change in the coil 32. Once this occurs, the control unit may switch the device to the resistive mode 60.

This change may be a change in impedance or resistance. Impedance is a measure of the amount of opposition that a part of a circuit may present to a current. For example, if part of a circuit is more resistive, the impedance indicates the level of resistance. If part of a circuit becomes more resistive over time, a change in impedance can indicate that corresponding change in resistance.

Because of this, a change in impedance or resistance is a suitable parameter to measure to determine when to switch the device from the RF mode to the resistive mode. With RF signal, over time the blood may start to char in the local zone of the RF signal. This charring may cause the blood to coagulate on the coil, inhibiting its ability to deliver a sufficient RF signal for vessel ablation and decreasing its conductance. Once this change is detected, the device may switch to the resistive mode. This may involve transmitting a current through the coil 32 when the at least one change is detected to heat the body vessel and the vessel wall 20.

As described above in Fig. 3, in the resistive mode 60 a power supply 54 may generate a current to transmit through the coil 32. The current may be transmitted through the first circuit, including the power supply 54, the first wire 34, the coil 32, and the second wire 36. As the current flows through the higher resistance part of the coil 32, the coil heats up. This may heat the vessel wall 20, causing further coagulation and occlusion than already occurred with the RF signal. Having these two modes and corresponding structures in one device allows a user to have the advantages of RF heating and resistive heating to occlude a body vessel.

Additionally or alternatively, the resistive mode 60 may involve using the RF generator. In this case, the resistive mode 60 may not need or use the power supply. Instead, once blood begins to char on the coil and isolate it from the body vessel, the RF signal will primarily heat the electrode resistively. This will automatically start the resistive mode 60 based on the overall change in resistance and/or impedance in the overall circuit. The balance between the RF mode and the resistive mode can be measured with a resistivity and/or impedance sensor. A constant power output can be maintained by adjusting a voltage source.

Returning to Fig. 5, the first wire 34 may be attached via a first lead 62 to a power supply 54 (e.g. a battery). Additionally, the second wire 36 may be attached to the power supply 54 through a second lead 64. Various conductive wires and connectors may be used to electrically couple parts of the device.

Fig. 6 depicts a method to fully occlude a body vessel 12. In step 66, the device may be positioned in the body vessel 12 adjacent the treatment site. At this time, blood flow 16 will be substantially normal. In step 68, the device may generate the RF signal with a signal generator. The RF signal may be transmitted with a monopolar system or a bipolar system. With the monopolar system, the RF signal may flow through the second circuit. In the bipolar mode, the RF signal may flow through the third circuit.

When the RF signal is transmitted, the method may include heating a local zone of the body vessel 12 after the step of transmitting the RF signal. This step of heating may cause swelling of the body vessel 12 at the treatment site, as depicted in step 68. Advantageously, the coil itself may maintain a first temperature near body temperature. As shown, the step of transmitting the RF signal may comprise avoiding contact of the coil 32 with the vessel wall 12. Alternatively, transmitting the RF signal may include contacting the coil 32 with the vessel wall 12, as such contact may facilitate vessel closure.

In step 70, charred blood 18 may start to coagulate and occlude the function of the coil in the RF mode. As the charred blood 18 coagulates on the coil, the device may detect at least one change in the coil. This change may be a change in impedance, a change in temperature, a change in time, a change in current, and a change in resistance. If the device (e.g. sensor) detects a change in impedance due to the charred blood 18, the device may shut off the RF signal and switch to the resistive mode.

Due to the charred blood 18, the device may no longer optimally heat and occlude the body vessel 12. In this condition, it may be advantageous to switch the device to a resistive mode. In step 72, the device may now generate the current with the power supply. The current may flow through the first circuit. As the vessel wall 12 swells, the step of transmitting a current through the coil may comprise contacting the vessel wall with the coil in the resistive mode. Because the resistive mode causes the coil itself to raise in temperature, direct contact with the vessel wall 12 may be advantageous. Heating the vessel wall with the current may comprise the heating swelling the vessel wall further.

In step 74, the resistive mode may start to fully occlude the body vessel 12. As such, the device may start to withdraw from the body vessel 12. This withdrawal may be manual or automatic. In step 76, the body vessel has been fully included with occlusion 22. The device may be fully withdrawn.

It should be understood that the foregoing relates to exemplary embodiments of the disclosure and that modifications may be made without departing from the scope of the following claims. While the disclosure has been described with respect to certain embodiments it will be appreciated that modifications and changes may be made by those skilled in the art.

**EP 3 199 119 A1**

**Claims**

1. A device to heat a body vessel in a body, the device comprising:

   a support comprising a proximal end and extending to a distal end, the support defining a longitudinal axis;
   a coil disposed about the support and being electrically conductive, the coil having a first end being disposed between the proximal and distal ends, the coil extending from the first end to a second end disposed at the distal end, the coil including a resistive part; and
   a control unit being operatively coupled to the first end and the second end, the control unit having a signal generator and a power supply, the signal generator operable to transmit a radiofrequency signal from the coil to the body vessel when the device is in a radiofrequency mode, the resistive part being at a first temperature in the radiofrequency mode, the power supply operable to transmit a current through the coil when the device is in a resistive mode, the current heating the resistive part to a second temperature greater than the first temperature and sufficient to occlude the body vessel.

2. The device of claim 1 wherein the support has a distal segment about which the coil is disposed, the distal segment having an outer diameter that distally decreases to form a distal taper.

3. The device of claim 1 wherein the support has an outer diameter being uniform from the proximal end to the distal end.

4. The device of any one of the preceding claims wherein the distal end forms an atraumatic tip.

5. The device of any one of the preceding claims further comprising an outer sheath having a first sheath end and extending to a second sheath end, the outer sheath forming an inner lumen therethrough from the first sheath end to the second sheath end, the support being slidably disposed within the inner lumen.

6. The device of any one of the preceding claims further comprising a first wire and a second wire, the first wire being electrically coupled to the control unit and extending from the control unit and along the longitudinal axis to the first end, the first wire being attached to the first end, the second wire also being electrically coupled to the control unit and extending from the control unit and along the longitudinal axis to the second end, the second wire being attached to the second end, the control unit, the first wire, the coil, and second wire forming a first circuit operable in the resistive mode.

7. The device of claim 6 having an insulator disposed about the first and second wires to electrically insulate them from the rest of the device.

8. The device of claim 6 further comprising a return electrode being electrically coupled to the control unit and disposable outside of the body, the signal generator, first wire, the coil, and return electrode forming a second circuit for the radiofrequency signal.

9. The device of claim 6 wherein the second wire comprises a return electrode being electrically coupled to the control unit and operable in the radiofrequency mode, the signal generator, the first wire, the coil, and the return electrode forming a third circuit for the radiofrequency signal.

10. The device of any one of the preceding claims further comprising a plurality of sensors coupled to the coil, the plurality of sensors being sensors selected from the group consisting of temperature sensors, current sensors, timers, impedance sensors, and resistance sensors.

11. The device of any one of the preceding claims further comprising shrink tubing disposed about the support, the coil being free from the shrink tubing.

7

FIG. 1

FIG. 2A

FIG. 2B

FIG. 3

FIG. 4

ESU
RF
Generator

FIG. 5

EP 3 199 119 A1

FIG. 6

Europäisches
Patentamt

European
Patent Office

Office européen
des brevets

## EUROPEAN SEARCH REPORT

Application Number

EP 17 15 1046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/161474 A1 (EMCISION LTD [GB]; HABIB NAGY [GB]) 29 December 2011 (2011-12-29) * abstract; figures 1-24 * * page 1, lines 5-10, 33-34 * * page 2, line 25 - page 9, line 29 * * page 12, lines 29-36 * * page 19, line 6 - page 21, line 18 * * page 27, lines 20-29 * | 1-11 | INV. A61B18/12 |
| A | US 2002/095151 A1 (DAHLA ROBERT H [US] ET AL) 18 July 2002 (2002-07-18) * abstract; figures 1-25 * * paragraphs [0003], [0008], [0012] - [0028], [0068], [0069], [0078] - [0080], [0084], [0089], [0127], [0157] * | 1-11 | |
| A | WO 2007/014063 A2 (VNUS MED TECH INC [US]; ESCH BRADY [US]; FARLEY BRIAN E [US]; THOMPSON) 1 February 2007 (2007-02-01) * abstract; figures 1-36 * * paragraphs [0005] - [0179], [0250], [0251], [0301], [0319], [0320], [0518], [0570], [0571] * | 1-10 | TECHNICAL FIELDS SEARCHED (IPC) A61B |
| A | WO 2006/049970 A2 (CARMEL YUVAL [US]; WAKSMAN RON [US]; SHKVARUNETS ANATOLY [US]) 11 May 2006 (2006-05-11) * abstract; figures 1-13 * * page 1, lines 5-13 * * page 3 - page 7 * * page 20, lines 13-20 * | 1 | |

The present search report has been drawn up for all claims

| Place of search | Date of completion of the search | Examiner |
|---|---|---|
| The Hague | 26 June 2017 | Moualed, Laura |

## ANNEX TO THE EUROPEAN SEARCH REPORT
## ON EUROPEAN PATENT APPLICATION NO.

EP 17 15 1046

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2017

| Patent document cited in search report | | Publication date | Patent family member(s) | | Publication date |
|---|---|---|---|---|---|
| WO 2011161474 | A1 | 29-12-2011 | CN | 103025261 A | 03-04-2013 |
| | | | EP | 2584987 A1 | 01-05-2013 |
| | | | US | 2013211176 A1 | 15-08-2013 |
| | | | WO | 2011161474 A1 | 29-12-2011 |
| US 2002095151 | A1 | 18-07-2002 | AT | 524128 T | 15-09-2011 |
| | | | EP | 1404236 A1 | 07-04-2004 |
| | | | US | 2002095151 A1 | 18-07-2002 |
| | | | US | 2002099366 A1 | 25-07-2002 |
| | | | US | 2007149966 A1 | 28-06-2007 |
| | | | US | 2008004621 A1 | 03-01-2008 |
| | | | WO | 02102255 A1 | 27-12-2002 |
| WO 2007014063 | A2 | 01-02-2007 | CN | 101355912 A | 28-01-2009 |
| | | | CN | 102225023 A | 26-10-2011 |
| | | | CN | 102225024 A | 26-10-2011 |
| | | | CN | 102389332 A | 28-03-2012 |
| | | | CN | 102389333 A | 28-03-2012 |
| | | | DE | 202006021213 U1 | 08-11-2013 |
| | | | DE | 202006021214 U1 | 08-11-2013 |
| | | | DE | 202006021215 U1 | 08-11-2013 |
| | | | EP | 1906853 A2 | 09-04-2008 |
| | | | EP | 2444018 A2 | 25-04-2012 |
| | | | EP | 2662040 A2 | 13-11-2013 |
| | | | JP | 5030180 B2 | 19-09-2012 |
| | | | JP | 2009504203 A | 05-02-2009 |
| | | | US | 2007049999 A1 | 01-03-2007 |
| | | | US | 2007050000 A1 | 01-03-2007 |
| | | | US | 2007055326 A1 | 08-03-2007 |
| | | | US | 2007055327 A1 | 08-03-2007 |
| | | | US | 2007100405 A1 | 03-05-2007 |
| | | | US | 2007179575 A1 | 02-08-2007 |
| | | | US | 2010106150 A1 | 29-04-2010 |
| | | | US | 2010114085 A1 | 06-05-2010 |
| | | | US | 2010145327 A1 | 10-06-2010 |
| | | | US | 2011034922 A1 | 10-02-2011 |
| | | | US | 2011046617 A1 | 24-02-2011 |
| | | | US | 2011054456 A1 | 03-03-2011 |
| | | | US | 2012071873 A1 | 22-03-2012 |
| | | | US | 2014378970 A1 | 25-12-2014 |
| | | | WO | 2007014063 A2 | 01-02-2007 |
| WO 2006049970 | A2 | 11-05-2006 | US | 2006089638 A1 | 27-04-2006 |
| | | | WO | 2006049970 A2 | 11-05-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62279061 A